# EUROPEAN PATENT APPLICATION

(11) **EP 0 839 909 A1**
(43) Date of publication of application: **06.05.1998**
(21) Application number: 96203015.1
(22) Date of filing: 29.10.1996
(51) Int. Cl.: C12N 15/31, C07K 14/32, C12N 1/21, C22B 3/18

(54) **Nucleic acid sequences encoding citrate transporter proteins**

(71) Applicant: Rijksuniversiteit te Groningen, 9712 CP Groningen (NL)
(72) Inventor: Konings, Wilhelmus Nicolaas, 9752 CJ Haren (NL); Boorsma, Andries, 9718 BJ Groningen (NL); Lolkema, Julius Sjieuwke, 9751 BH Laren (NL)
(74) Representative: Smulders, Theodorus A.H.J., Ir.

(57) **Abstract**

The invention relates to nucleic acids that encode a secondary transporter that transports citrate or the complex of citrate and metal ions or metal salt ions. These nucleic acids can be functionally expressed in host cells such as *E. coli.*and *Bacillus*, or other host cells. The transporter proteins encoded by the nucleic acids of the invention and host cells or biological membranes comprising the proteins facilitate and enhance the removal of heavy metals from compositions such as waste, waste disposal sites, metal ores.

SEQUENCE LISTING

(1) GENERAL INFORMATION:

(i) APPLICANT:
(A) NAME: Rijksuniversiteit Groningen
(B) STREET: Broerstraat 5
(C) CITY: Groningen
(D) STATE: Groningen
(E) COUNTRY: the Netherlands
(F) POSTAL CODE (ZIP): 9712 CP

(ii) TITLE OF INVENTION: Nucleic acid sequences encoding citrate
transporter proteins.

(iii) NUMBER OF SEQUENCES: 11

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96203015.1

(2) INFORMATION FOR SEQ ID NO: 1:

(i) SEQUENCE CHARACTERISTICS:
(A) LENGTH: 24 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 1:

TTAAGGGGCC ATGGATGTGT TAGC 24

(2) INFORMATION FOR SEQ ID NO: 2:

(i) SEQUENCE CHARACTERISTICS:
(A) LENGTH: 20 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 2:

CTCCCCAAGG AATCGTGTTC 20

(2) INFORMATION FOR SEQ ID NO: 3:

(i) SEQUENCE CHARACTERISTICS:
(A) LENGTH: 36 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 3:

ATGAATCTTC TAGACACTCA TAGGATCCTA TTGATC 36

(2) INFORMATION FOR SEQ ID NO: 4:

(i) SEQUENCE CHARACTERISTICS:
(A) LENGTH: 26 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 4:

GTCATTACGC CTGAATTCCT CATACG 26

(2) INFORMATION FOR SEQ ID NO: 5:

(i) SEQUENCE CHARACTERISTICS:
(A) LENGTH: 44 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 5:

AAAAAAGCTT TTGAATAGGG GAGGTCATAC CATGGTTGCC ATAC 44

(2) INFORMATION FOR SEQ ID NO: 6:

(i) SEQUENCE CHARACTERISTICS:
(A) LENGTH: 1314 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 6:

GGGGGAATGG ATGTGTTAGC AATCTTAGGC TTTCTCATGA TGCTTGTGTT TATGGCATTG 60

ATCATGACAA AACGGCTTTC TGTTTTAACA GCATTAGTTT TGACGCCGAT TGTGTTTGCG 120

CTTATCGCCG GATTTGGATT TACTGAAGTT GGGGACATGA TGATTTCGGG GATTCAGCAA 180

GTCGCGCCGA CTGCGGTCAT GATTATGTTT GCGATCTTAT ATTTTGGAAT TATGATTGAT 240

ACAGGCCTGT TTGATCCAAT GGTTGGCAAA ATTTTAAGCA TGGTCAAAGG AGACCCTTTA 300

AAAATTGTTG TCGGGACAGC GGTTCTTACA ATGCTCGTCG CCTTGGACGG AGATGGCTCG 360

ACAACGTACA TGATTACGAC AAGCGCCATG CTTCCGCTCT ATTTGCTGCT AGGCATCCGG 420

CCAATTATCT TGGCAGGAAT CGCGGGAGTC GGCATGGGAA TCATGAACAC GATTCCATGG 480

GGAGGTGCGA CGCCGAGGGC GGCGAGTGCG CTGGGGGTTG ATCCAGCTGA GCTTACAGGG 540

CCGATGATTC CTGTCATTGC AAGCGGGATG CTTTGTATGG TGGCAGTTGC GTATGTGCTT 600

GGAAAAGCGG AACGAAAGCG CCTTGGTGTG ATTGAACTGA AACAGCCAGC CAATGCCAAT 660

GAACCGGCTG CTGCGGTTGA AGATGAGTGG AAGCGGCCGA AGCTTTGGTG GTTCAATTTA 720

TTGTTAACGC TTTCTTTAAT AGGATGTTTA GTATCGGGTA AAGTCAGTTT AACCGTACTG 780

TTTGTCATTG CGTTTTGTAT TGCGCTGATT GTGAATTATC CCAATCTCGA GCATCAGAGA 840

CAGCGCATCG CGGCGCATTC CAGCAACGTG CTGGCTATCG GTTCAATGAT TTTTGCTGCG 900

GGGGTGTTCA CGGGGATTTT GACAGGCACG AAAATGGTTG ATGAAATGGC GATCTCGCTC 960

GTGTCCATGA TACCGGAACA AATGGGCGGA TTGATCCCGG CGATTGTTGC CTTAACAAGC 1020

GGCATTTTCA CATTTTTGAT GCCGAATGAC GCGTATTTCT ACGGGGTGCT GCCGATTTTA 1080

TCAGAAACAG CTGTCGCATA CGGTGTGGAT AAAGTGGAAA TTGCCAGAGC CTCTATTATC 1140

GGCCAGCCGA TTCATATGCT GAGTCCGCTT GTGCCATCCA CTCATTTGCT TGTCGGACTC 1200

GTCGGAGTTT CTATTGATGA CCATCAAAAA TTCGCATTGA AATGGGCGGT TCTCGCAGTG 1260

ATCGTCATGA CGGCTATCGC TCTATTGATC GGTGCGATCT CTATTTCCGT ATGA 1314

(2) INFORMATION FOR SEQ ID NO: 7:

(i) SEQUENCE CHARACTERISTICS:
(A) LENGTH: 435 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 7:

Met Asp Val Leu Ala Ile Leu Gly Phe Leu Met Met Leu Val Phe Met
1               5                   10                  15

Ala Leu Ile Met Thr Lys Arg Leu Ser Val Leu Thr Ala Leu Val Leu
            20                  25                  30

Thr Pro Ile Val Phe Ala Leu Ile Ala Gly Phe Gly Phe Thr Glu Val
        35                  40                  45

Gly Asp Met Met Ile Ser Gly Ile Gln Gln Val Ala Pro Thr Ala Val
    50                  55                  60

Met Ile Met Phe Ala Ile Leu Tyr Phe Gly Ile Met Ile Asp Thr Gly
65                  70                  75                  80

Leu Phe Asp Pro Met Val Gly Lys Ile Leu Ser Met Val Lys Gly Asp
                85                  90                  95

Pro Leu Lys Ile Val Val Gly Thr Ala Val Leu Thr Met Leu Val Ala
            100                 105                 110

Leu Asp Gly Asp Gly Ser Thr Thr Tyr Met Ile Thr Thr Ser Ala Met
        115                 120                 125

Leu Pro Leu Tyr Leu Leu Leu Gly Ile Arg Pro Ile Ile Leu Ala Gly
    130                 135                 140

Ile Ala Gly Val Gly Met Gly Ile Met Asn Thr Ile Pro Trp Gly Gly
145                 150                 155                 160

Ala Thr Pro Arg Ala Ala Ser Ala Leu Gly Val Asp Pro Ala Glu Leu
                165                 170                 175

Thr Gly Pro Met Ile Pro Val Ile Ala Ser Gly Met Leu Cys Met Val
            180                 185                 190

Ala Val Ala Tyr Val Leu Gly Lys Ala Glu Arg Lys Arg Leu Gly Val
        195                 200                 205

Ile Glu Leu Lys Gln Pro Ala Asn Ala Asn Glu Pro Ala Ala Ala Val
    210                 215                 220

Glu Asp Glu Trp Lys Arg Pro Lys Leu Trp Trp Phe Asn Leu Leu Leu
225                 230                 235                 240

Thr Leu Ser Leu Ile Gly Cys Leu Val Ser Gly Lys Val Ser Leu Thr
                245                 250                 255

Val Leu Phe Val Ile Ala Phe Cys Ile Ala Leu Ile Val Asn Tyr Pro
            260                 265                 270

Asn Leu Glu His Gln Arg Gln Arg Ile Ala Ala His Ser Ser Asn Val
        275                 280                 285

Leu Ala Ile Gly Ser Met Ile Phe Ala Ala Gly Val Phe Thr Gly Ile
    290                 295                 300

Leu Thr Gly Thr Lys Met Val Asp Glu Met Ala Ile Ser Leu Val Ser
305                 310                 315                 320

Met Ile Pro Glu Gln Met Gly Gly Leu Ile Pro Ala Ile Val Ala Leu

325                 330                 335

Thr Ser Gly Ile Phe Thr Phe Leu Met Pro Asn Asp Ala Tyr Phe Tyr
            340                 345                 350

Gly Val Leu Pro Ile Leu Ser Glu Thr Ala Val Ala Tyr Gly Val Asp
        355                 360                 365

Lys Val Glu Ile Ala Arg Ala Ser Ile Ile Gly Gln Pro Ile His Met
    370                 375                 380

Leu Ser Pro Leu Val Pro Ser Thr His Leu Leu Val Gly Leu Val Gly
385                 390                 395                 400

Val Ser Ile Asp Asp His Gln Lys Phe Ala Leu Lys Trp Ala Val Leu
                405                 410                 415

Ala Val Ile Val Met Thr Ala Ile Ala Leu Leu Ile Gly Ala Ile Ser
            420                 425                 430

Ile Ser Val
        435

(2) INFORMATION FOR SEQ ID NO: 8:

(i) SEQUENCE CHARACTERISTICS:
(A) LENGTH: 1296 base pairs
(B) TYPE: nucleic acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 8:

AGGGGAGGTC ATATCATGCT TGCCATACTC GGTTTTGTGA TGATGATTGT CTTTATGTAC      60

CTTATTATGT CTAACCGGCT TTCCGCTCTT ATTGCTTTGA TTGTCGTTCC TATTGTGTTT     120

GCCCTGATCA GCGGATTTGG CAAAGATCTC GGCGAGATGA TGATTCAGGG CGTTACAGAC     180

CTCGCCCCTA CCGGTATCAT GCTGTTATTC GCCATCCTGT ATTTCGGCAT TATGATTGAC     240

TCAGGCCTGT TTGATCCTCT CATTGCCAAA ATCTTATCGT TTGTCAAAGG AGATCCGTTT     300

AAAATCGCCG TAGGCACAGC GGTTCTGACC ATGACCATTT CGCTGGACGG AGATGGGACA     360

ACAACCTATA TGATTACCAT TCGAGCGATG CTGCCTCTCT ACAAACGGCT CGGCATGAAC     420

CGTTTGGTGT TAGCGGGAAT AGCGATGCTT GGTTCGGGGG TTATGAATAT TATCCCGTGG     480

GGCGAGCCGA CTGCGAGGGT TTTGGCTTCC TTAAAATTGG ACACGTCAGA GGTCTTTACA     540

CCGCTGATTC CCGCTATGAT CGCCGGCATT CTCTGGGTGA TCGCCGTTGC TTATATCCTC     600

GGAAAGAAAG AGCGGAAGCG GCTCGGCGTC ATTTCGATTG ATCACGCACC GTCTTCCGAC     660

CCGGAGGCCG CACCGCTCAA GCGTCCCGCT CTTCAATGGT TTAACCTGCT GCTGACTGTC     720

GCTCTGATGG CCGCACTGAT CACCAGCCTG CTGCCGCTCC CTGTTCTTTT TATGACTGCG     780

TTCGCCGTTG CCTTGATGGT TAACTATCCA AATGTCAAAG AGCAGCAGAA ACGAATCTCG 840

GCGCATGCGG GTAATGCGTT AAACGTTGTC TCAATGGTGT TTGCTGCGGG CATATTCACA 900

GGCATTCTCT CCGGCACAAA AATGGTGGAT GCCATGGCGC ATTCTACACG TTCACTCATC 960

CCTGATGCCA TGGGCCCGCA CCTGCCGTTG ATCACTGCGA TCGTCAGCAT GCCCTTCACC 1020

TTTTTCATGT CGAATGACGC CTTTTACTTC GGTGTCCTTC CCATCATCGC CGAAGCCGCT 1080

TCCGCTTACG GAATAGACGC CGCTGAAATC GGGAGGGCCT CCTTGCTGGG GCAGCCTGTG 1140

CATCTGCTCA GCCCGCTTGT GCCTTCCACC TATCTATTGG TAGGAATGGC AGGCGTCAGC 1200

TTTGGCGACC ATCAAAAATT CACTATTAAA TGGGCCGTGG GAACAACGAT TGTGATGACC 1260

ATTGCGGCGC TTTTGATTGG GATTATTTCT TTCTAA 1296

(2) INFORMATION FOR SEQ ID NO: 9:

(i) SEQUENCE CHARACTERISTICS:
(A) LENGTH: 426 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 9:

Met Leu Ala Ile Leu Gly Phe Val Met Met Ile Val Phe Met Tyr Leu
1               5                   10                  15

Ile Met Ser Asn Arg Leu Ser Ala Leu Ile Ala Leu Ile Val Val Pro
            20                  25                  30

Ile Val Phe Ala Leu Ile Ser Gly Phe Gly Lys Asp Leu Gly Glu Met
        35                  40                  45

Met Ile Gln Gly Val Thr Asp Leu Ala Pro Thr Gly Ile Met Leu Leu
    50                  55                  60

Phe Ala Ile Leu Tyr Phe Gly Ile Met Ile Asp Ser Gly Leu Phe Asp
65                  70                  75                  80

Pro Leu Ile Ala Lys Ile Leu Ser Phe Val Lys Gly Asp Pro Phe Lys
                85                  90                  95

Ile Ala Val Gly Thr Ala Val Leu Thr Met Thr Ile Ser Leu Asp Gly
            100                 105                 110

Asp Gly Thr Thr Thr Tyr Met Ile Thr Ile Arg Ala Met Leu Pro Leu
        115                 120                 125

Tyr Lys Arg Leu Gly Met Asn Arg Leu Val Leu Ala Gly Ile Ala Met
    130                 135                 140

Leu Gly Ser Gly Val Met Asn Ile Ile Pro Trp Gly Glu Pro Thr Ala
145                 150                 155                 160

Arg Val Leu Ala Ser Leu Lys Leu Asp Thr Ser Glu Val Phe Thr Pro
                165                 170                 175

Leu Ile Pro Ala Met Ile Ala Gly Ile Leu Trp Val Ile Ala Val Ala
            180                 185                 190

Tyr Ile Leu Gly Lys Lys Glu Arg Lys Arg Leu Gly Val Ile Ser Ile
        195                 200                 205

Asp His Ala Pro Ser Ser Asp Pro Glu Ala Ala Pro Leu Lys Arg Pro
    210                 215                 220

Ala Leu Gln Trp Phe Asn Leu Leu Leu Thr Val Ala Leu Met Ala Ala
225                 230                 235                 240

Leu Ile Thr Ser Leu Leu Pro Leu Pro Val Leu Phe Met Thr Ala Phe
                245                 250                 255

Ala Val Ala Leu Met Val Asn Tyr Pro Asn Val Lys Glu Gln Gln Lys
            260                 265                 270

Arg Ile Ser Ala His Ala Gly Asn Ala Leu Asn Val Val Ser Met Val
        275                 280                 285

Phe Ala Ala Gly Ile Phe Thr Gly Ile Leu Ser Gly Thr Lys Met Val
    290                 295                 300

Asp Ala Met Ala His Ser Thr Arg Ser Leu Ile Pro Asp Ala Met Gly
305                 310                 315                 320

Pro His Leu Pro Leu Ile Thr Ala Ile Val Ser Met Pro Phe Thr Phe
                325                 330                 335

Phe Met Ser Asn Asp Ala Phe Tyr Phe Gly Val Leu Pro Ile Ile Ala
            340                 345                 350

Glu Ala Ala Ser Ala Tyr Gly Ile Asp Ala Ala Glu Ile Gly Arg Ala
        355                 360                 365

Ser Leu Leu Gly Gln Pro Val His Leu Leu Ser Pro Leu Val Pro Ser
    370                 375                 380

Thr Tyr Leu Leu Val Gly Met Ala Gly Val Ser Phe Gly Asp His Gln
385                 390                 395                 400

Lys Phe Thr Ile Lys Trp Ala Val Gly Thr Thr Ile Val Met Thr Ile
                405                 410                 415

Ala Ala Leu Leu Ile Gly Ile Ile Ser Phe
            420                 425

(2) INFORMATION FOR SEQ ID NO: 10:

     (i) SEQUENCE CHARACTERISTICS:
          (A) LENGTH: 426 amino acids
          (B) TYPE: amino acid
          (C) STRANDEDNESS: unknown
          (D) TOPOLOGY: unknown

    (ii) MOLECULE TYPE: protein

   (iii) HYPOTHETICAL: NO

(xi) SEQUENCE DESCRIPTION: SEQ ID NO: 10:

Met Leu Ala Ile Leu Gly Phe Val Met Met Ile Val Phe Met Tyr Leu
1               5                   10                  15

Ile Met Ser Asn Arg Leu Ser Ala Leu Ile Ala Leu Ile Val Val Pro
            20                  25                  30

Ile Val Phe Ala Leu Ile Ser Gly Phe Gly Lys Asp Leu Gly Glu Met
        35                  40                  45

Met Ile Gln Gly Val Thr Asp Leu Ala Pro Thr Gly Ile Met Leu Leu
    50                  55                  60

Phe Ala Ile Leu Tyr Phe Gly Ile Met Ile Asp Ser Gly Leu Phe Asp
65                  70                  75                  80

Pro Leu Ile Ala Lys Ile Leu Ser Phe Val Lys Gly Asp Pro Phe Lys
                85                  90                  95

Ile Ala Val Gly Thr Ala Val Leu Thr Met Thr Ile Ser Leu Asp Gly
            100                 105                 110

Asp Gly Thr Thr Thr Tyr Met Ile Thr Ile Arg Ala Met Leu Pro Leu
        115                 120                 125

Tyr Lys Arg Leu Gly Met Asn Arg Leu Val Leu Ala Gly Ile Ala Met
    130                 135                 140

Leu Gly Ser Gly Val Met Asn Ile Ile Pro Trp Gly Glu Pro Thr Ala
145                 150                 155                 160

Arg Val Leu Ala Ser Leu Lys Leu Asp Thr Ser Glu Val Phe Thr Pro
                165                 170                 175

Leu Ile Pro Ala Met Ile Ala Gly Ile Leu Trp Val Ile Ala Val Ala
            180                 185                 190

Tyr Ile Leu Gly Lys Lys Glu Arg Lys Arg Leu Gly Val Ile Ser Ile
        195                 200                 205

Asp His Ala Pro Ser Ser Asp Pro Glu Ala Ala Pro Leu Lys Arg Pro
    210                 215                 220

Ala Leu Gln Trp Phe Asn Leu Leu Leu Thr Val Ala Leu Met Ala Ala
225                 230                 235                 240

Leu Ile Thr Ser Leu Leu Pro Leu Pro Val Leu Phe Met Thr Ala Phe
                245                 250                 255

Ala Val Ala Leu Met Val Asn Tyr Pro Asn Val Lys Glu Gln Gln Lys
            260                 265                 270

Arg Ile Ser Ala His Ala Gly Asn Ala Leu Asn Val Val Ser Met Val
        275                 280                 285

Phe Ala Ala Gly Ile Phe Thr Gly Ile Leu Ser Gly Thr Lys Met Val
    290                 295                 300

Asp Ala Met Ala His Ser Thr Arg Ser Leu Ile Pro Asp Ala Met Gly
305                 310                 315                 320

Pro His Leu Pro Leu Ile Thr Ala Ile Val Ser Met Pro Gly Thr Phe
                325                 330                 335

Phe Met Ser Asn Asp Ala Phe Tyr Phe Gly Val Leu Pro Ile Ile Ala
            340                 345                 350

Glu Ala Ala Ser Ala Tyr Gly Ile Asp Ala Ala Glu Ile Gly Arg Ala
        355                 360                 365

Ser Leu Leu Gly Gln Pro Val His Leu Leu Ser Pro Leu Val Pro Ser
    370                 375                 380

Thr Tyr Leu Leu Val Gly Met Ala Gly Val Ser Phe Gly Asp His Gln
385                 390                 395                 400

Lys Phe Thr Ile Lys Trp Ala Val Gly Thr Thr Ile Val Met Thr Ile
                405                 410                 415

Ala Ala Leu Leu Ile Gly Ile Ile Ser Phe
            420                 425

(2) INFORMATION FOR SEQ ID NO: 11:

    (i) SEQUENCE CHARACTERISTICS:
        (A) LENGTH: 437 amino acids
        (B) TYPE: amino acid
        (C) STRANDEDNESS: unknown
        (D) TOPOLOGY: unknown

   (ii) MOLECULE TYPE: protein

  (iii) HYPOTHETICAL: NO

   (xi) SEQUENCE DESCRIPTION: SEQ ID NO: 11:

Met Asp Val Leu Ala Ile Leu Gly Phe Leu Met Met Leu Val Phe Met
1               5                   10                  15

Ala Leu Ile Met Thr Lys Arg Leu Ser Val Leu Thr Ala Leu Val Leu
            20                  25                  30

Thr Pro Ile Val Phe Ala Leu Ile Ala Gly Phe Gly Phe Thr Glu Val
        35                  40                  45

Gly Asp Met Met Ile Ser Gly Ile Gln Gln Val Ala Pro Thr Ala Val
    50                  55                  60

Met Ile Met Phe Ala Ile Leu Tyr Phe Gly Ile Met Ile Asp Thr Gly
65                  70                  75                  80

Leu Phe Asp Pro Met Val Gly Lys Ile Leu Ser Met Val Lys Gly Asp
                85                  90                  95

Pro Leu Lys Ile Val Val Gly Thr Ala Val Leu Thr Met Leu Val Ala
            100                 105                 110

Leu Asp Gly Asp Gly Ser Thr Thr Tyr Met Ile Thr Thr Ser Ala Met
        115                 120                 125

Leu Pro Leu Tyr Leu Leu Leu Gly Ile Arg Pro Ile Ile Leu Ala Gly
    130                 135                 140

Ile Ala Gly Val Gly Met Gly Ile Met Asn Thr Ile Pro Trp Gly Gly
145                 150                 155                 160

Ala Thr Pro Ser Gly Ser Ala Leu Gly Val Asp Pro Ala Glu Leu Thr
                165                 170                 175

Gly Pro Met Ile Pro Val Ile Ala Ser Gly Met Leu Cys Met Val Ala
            180                 185                 190

Val Ala Tyr Val Leu Gly Lys Ala Glu Arg Lys Arg Leu Gly Val Ile
        195                 200                 205

Glu Leu Lys Gln Pro Ala Asn Ala Asn Glu Pro Ala Ala Ala Val Glu
    210                 215                 220

Asp Glu Trp Lys Pro Ala Lys Leu Trp Trp Phe Asn Leu Leu Leu Thr
225                 230                 235                 240

Leu Ser Leu Ile Gly Cys Leu Val Ser Gly Lys Val Ser Leu Thr Val
                245                 250                 255

Leu Phe Val Ile Ala Phe Cys Ile Ala Leu Ile Val Asn Tyr Pro Asn
            260                 265                 270

Leu Glu His Gln Arg Gln Arg Ile Ala Ala His Ser Ser Asn Val Leu
        275                 280                 285

Ala Ile Gly Ser Met Ile Phe Ala Ala Gly Val Phe Thr Gly Ile Leu
    290                 295                 300

Thr Gly Thr Lys Met Val Asp Glu Met Ala Ile Ser Leu Val Ser Met
305                 310                 315                 320

Ile Pro Glu Gln Met Gly Gly Leu Ile Pro Ala Ile Val Ala Leu Thr
                325                 330                 335

Ser Gly Ile Phe Thr Phe Leu Met Pro Asn Asp Ala Tyr Phe Tyr Gly
            340                 345                 350

Val Leu Pro Ile Leu Ser Glu Thr Ala Val Ala Tyr Gly Val Asp Lys
        355                 360                 365

Val Glu Ile Ala Arg Ala Ser Ile Ile Gly Gln Pro Ile His Met Leu
    370                 375                 380

Ser Pro Leu Val Pro Ser Thr His Leu Leu Val Gly Leu Val Gly Leu
385                 390                 395                 400

Val Gly Val Ser Ile Asp Asp His Gln Lys Phe Ala Leu Lys Trp Ala
                405                 410                 415

Val Leu Ala Val Ile Val Met Thr Ala Ile Ala Leu Leu Ile Gly Ala
            420                 425                 430

Ile Ser Ile Ser Val
        435

## Description

Heavy metals are becoming an increasingly serious problem to the environment. Their toxicity and the fact that they tend to get diluted or dissolved and get distributed from out of waste deposits make them a major environmental hazard, whereas at the same time those same metals compose a precious commodity which would make it worthwhile to harvest these metals from the environment. Environmental contamination with metals occurs for example in waste, or in ground or process water stemming from mining, the metal industry, viscose industry, rubber industry, paper industry, potato industry, starch processing, yeast industry, and so on. In addition, mining for metals in itself is a process where metals are harvested from the environment, and all above methods entail processes in which metals are recovered.

Bacterial or biological technology, using selected naturally occuring microorganisms that can help concentrate and accumulate heavy metals, is currently in development. Sulphate reducing bacterial strains have been detected in nature which specifically convert sulphate into sulphite (which takes place under oxygen-free, anaerobic conditions). The sulphite than reacts with dissolved metals, producing a metal precipitate which can be removed. Such processes can be carried out in reactors. Other biological processes for recovering metals make use of bacteria that reduce the heavy metal itself and create metal precipitates, however, the above described biological processes are basically performed in an oxygen-free environment under anaerobic conditions, conditions which are not easy to achieve when large masses of waste or material containing dilute concentrations of the sought-after metal need to be handled.

Citrate is very abundant in nature and most bacteria have transport proteins in the cytoplasmic membrane that mediate the uptake of citrate. The carriers belong to the class of secondary transporters that use the free energy stored in transmembrane electrochemical gradients of ions to drive the uptake of the substrates (for a review, see 18). The citrate transporter CitH of *Klebsiella pneumoniae* is driven by the proton motive force (23) and the transporters CitS and CitC of *K. pneumoniae* and *Salmonella serovars* by both the proton motive force and sodium ion motive force (9,14,19,24). Mechanistically these transporters catalyze coupled translocation of citrate and H⁺ and/or Na⁺ (symport). A special case are the citrate carriers of lactic acid bacteria that take up citrate by an electrogenic uniport mechanism or by exchange with lactate, a product of citrate metabolism (citrolactic fermentation) (16,17,20). These citrate transporters are involved in secondary metabolic energy generation (12).

A number of structural genes coding for citrate transporters have been cloned and the primary sequences have been deduced from the base sequences. The proton dependent citrate carrier of *K. pneumoniae* CitH belongs to a large family of proteins in which also many sugar transporters are found (22). The Na⁺ dependent citrate carriers CitS of *K. pneumoniae* and CitC of *S. serovars* form together with the citrate carriers of lactic acid bacteria CitP's a distinct family termed the 2-hydroxy-carboxylate carriers (4,15,24). The malate transporter of *Lactococcus lactis* MleP that is involved in malolactic fermentation is also a member of this family (1).

Citrate is a chelator that forms stable complexes with various metal ions or metalsalt ions such as but not limited to Mn²⁺, Zn²⁺, Mg²⁺, Be²⁺, Ba²⁺, Ca²⁺, Cu²⁺, Co²⁺, Fe²⁺, Fe³⁺, Pb²⁺, Cd²⁺, UO₂²⁺, and Ni²⁺. The presence of metal ions resuits in inhibition of citrate transport activity by the transporters mentioned above (16,23,24) showing that the metal ion/citrate complex is not a substrate of these citrate transporters. On the other hand, other bacteria including *Pseudomonas* and *Klebsiella* spp and *Bacillus subtilis* are known to preferentially take up and degrade citrate in the metal ion bound complex (2,3,10,26).

These microorganisms have been implicated in the prevention of mobilization of toxic metal wastes by chelators like citrate. Degradation of the metal ion/citrate complex would render the metal ion in an insoluble, immobilized state (7). A complication is that the nature of the metal ion in the complex determines whether or not the complex is degraded. Studies with *Pseudomonas fluorescens* have shown that at least for a number of metal ions the lack of degradation was limited by the lack of transport of the complex into the cells and not because of the toxicity of the metal ion. The transporter seemed to recognize only the bidentate metal ion/citrate complexes that leave the hydroxyl group of citrate free; and not tridentate complexes (10).

As an example, the citrate carrier or citrate transporter protein of *B. subtilis* transports citrate in a complex with a wide variety of metal ions. Studies with membrane vesicles showed that the highest uptake rates were observed with Mn²⁺, intermediate rates with Zn²⁺, Mg²⁺, Be²⁺, Ba²⁺, Ca²⁺ and Cu²⁺ and the lowest rates with Co²⁺ and Ni²⁺ (2). It can however be expected that other citrate carrier have other metal specificities.

The present invention now provides a nucleic acid and derivatives thereof encoding genes of a new family of secondary transporter proteins. A first gene, termed CitM, was identified in *Bacillus subtilis*. Functional expression in *Escherichia coli* showed this gene to encode a citrate transporter protein that preferentially transports a metal-citrate complex. The invention now thus provides a gene that can be introduced in any bacterial or biological host cell to be used in bacterial or biological processing or recovery of metal. Such host cells can for example be selected from any of the bacterial strains from the genera *Pseudomonas, Klebsiella*, or *Bacillus*, but many other bacteria or other host cells can also be used. One may for example select those strains that thrive well in the presence of metal or metalsalt ions of various nature. A second gene, termed CitH, was identified in *Bacillus subtilis,* by searching of available databases for protein sequences resembling the CitM gene. The invention thus reveals a further citrate transporter.

The invention thus provides genes that code for a new family of secondary transporters. Studying the topology of the proteins encoded by these genes reveals a protein topology consisting of 12 transmembrane segments interspaced by a total of 11 loops. Within these transmembrane fragments and loops topology requirements and active sites are present which, when modified via recombinant technology, will alter the metal specificity of the encoded citrate transporter protein. Thus modified transporter proteins have either a narrower or broader metal ion specificity in the metal ion/citrate complex that is recognized by the carrier. The present invention encompasses this possibility of generating a nucleic acid sequence encoding a modified citrate transporter protein via the further application of recombinant DNA technology. Such modifications may be single or multiple mutations, substitutions, deletions or insertions or combinations thereof that can be achieved via any recombinant DNA technology methods known in the art. The present invention makes it possible to modify citrate transporter proteins that can be used to specifically interact with a particular metal or metals or a salt thereof, and can be used in processes recovering metals. Such taylor made proteins can be used *in vivo*, as being present as the active component in bacteria used for the recovery of metals from industrial waste and the like, but can also be used *in vitro* as active component in for example artificial biomembranes that will be used for metal recovery.

The invention also provides methods to select microorganisms that comprise said genes of a new family of secondary transporter proteins. Such selection methods can for example be based on a wide array of nucleic acid amplification techniques that is now available to the art. Such microorganisms can than advantagously be used for the industrial recovery of metal.

### Experimental part

### MATERIALS AND METHODS

### Bacterial strains and growth conditions.

*E. coli* strain JM101 harboring plasmid pWSKcitM coding for the divalent cation dependent citrate carrier of *B. subtilis* and strain JM109(DE3) harboring plasmid pWSKcitH coding for the proton dependent citrate carrier of *B. subtilis* were grown in 6 l flasks containing 1 l medium at 37 °C and under vigorous shaking. JM101 was grown in LB medium, JM109(DE3) in LB medium or minimal medium containing citrate as the sole carbon source. Carbenicillin was added at a concentration of 100 µg/ml and isopropyl-β-D-thiogalactopyranoside (IPTG) was added when appropriate. The cells were harvested in the late exponential growth phase and used immediately for uptake experiment or the preparation of membrane vesicles. *B. subtilis* strain 6GM was grown at 37 °C with vigorous aeration in medium containing 0.8% trypton (Difco), 0.5% NaCl, 25 mM KCl and 10 mM Na-citrate.

### Cloning and sequencing of CitM.

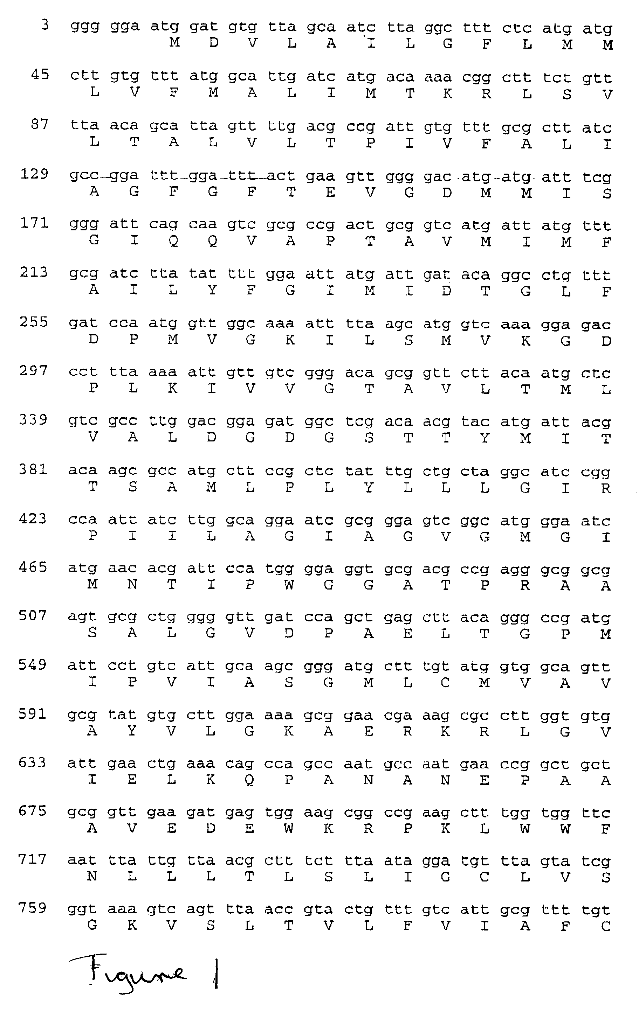

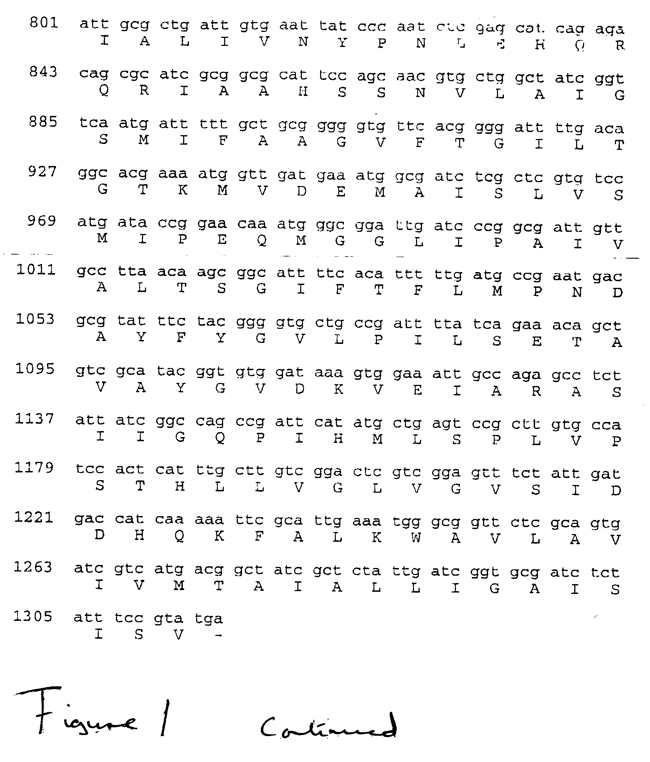

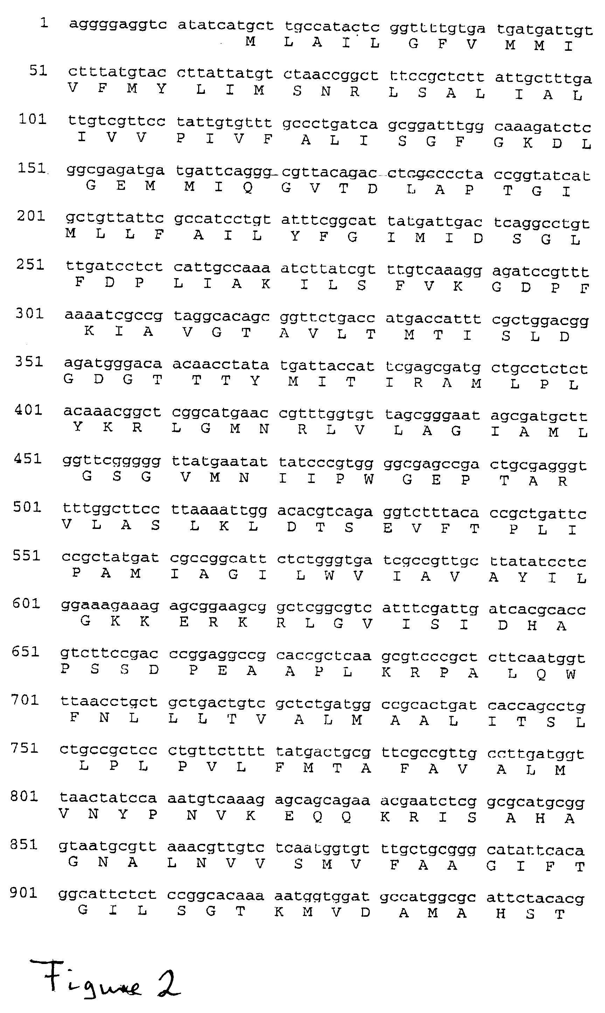

Chromosomal and plasmid DNA isolations and all other genetic techniques were done using the standard protocols described by Sambrook et al. (21) or the manufacturers protocols. Chromosomal DNA isolated from *B. subtilis* 6GM was partially digested with the restriction enzyme *Hind*III and the fragments were ligated in the multiple cloning site of vector pINIIIA (8) restricted with the same enzyme. Two fragments of 0.9 kb and 1.8 kb were restricted from clone pM5 by *Hind*III and ligated in the multiple cloning site of plasmid pBluescript II SK (Stratagene) yielding pSK0.9 and pSK1.8. Sets of nested deletions starting at both ends of the inserts were constructed of pSK0.9 and pSK1.8 using the Erase-a-base System (Promega). The plasmids were digested with *Kpn*I or *Sac*I to create protected 3' overhangs and *Sal*I or *Bam*HI to allow digestion into the fragments. The subclones were sequenced on a Vistra 725 automated sequencer using Texas Red labeled forward and reverse primers of the pBluescript vector (Fig. 1). The sequencing reactions were performed using the Thermo Sequenase labelled primer cycle sequencing kit (Vistra Systems) with 7-deaza-dGTP according to the manufacturers protocol.

### Southern blotting.

DNA probes were prepared by amplifying the regions on genomic DNA of *B. subtilis* that code for CitM and open reading frame N15CR by PCR. The CitM probe constitutes approximately the first 487 nucleotides of citM and the N15CR probe the last 463 nucleotides of N15CR. The probes were labeled with digoxygenin by including DIG-dUTP in the PCR reaction. The reaction contained in a total volume of 30 µl, 3 µl superTaq buffer, 10 ng template DNA, 6 µl of a mixture of dATP, dCTP, dGTP (0.65 mM each) and DIG-dUTP 0.35 mM, 2.5 U superTaq and 1 µl gelatine (5 mg/ml stock). The oligonucleotide primers were used at a concentration of 0.03 µg/ul. The forward and backward primers for the CitM probe were
5'- TTAAGGGGCCATGGA
TGTGTTAGC-3' and 5'-CTCCCCAAGGAATCGTGTTC-3' and for the N15CR probe 5'-GG
TGGATGCAATGGCGCATTC-3' and 5'-ATGAATCTTCTAGACACTCATAG-GATCCTATT
GATC-3'. The PCR reactions yielded fragments of the expected size and control reactions in the absence of DIG-UTP yielded identical fragments. Restriction analysis confirmed that the correct regions had been amplified. The DIG-dUTP labeled products were purified over an QiaQuick column (Qiagen) and approximately 1 µg of labeled probe was used per hybridization.
DNA was electrophoresed in agarose gels and blotted on Zeta-Probe Blotting Membrane (Biorad) using the Vacuum Blotting Unit (LKB Bromma). Sample preparation and transfer was essentially performed as described by Sambrook et al. (21). The blots were incubated with the labeled probes overnight at 65 °C. Subsequently, the membranes were washed twice with 300 mM NaCl, 30 mM Na-citrate, 0.1 % SDS for 5 min and at room temperature and twice with 15 mM NaCl, 1.5 mM Na-citrate, 0.1% SDS for 15 min at 65 °C. The membranes were used immediately for detection of hybridization.

### Construction of the expression vectors.

*pWSKcitM*. The citM gene was amplified by PCR using the Vent polymerase (Biolabs) from chromosomal DNA isolated from *B. subtilis* 6GM. The forward primer 5'-TTA*AGGG*GCCAT GGAT**GTG**TTAGC-3' contained the putative ribosomal binding site (indicated in italics) and the valine start codon (bold) and two mutations that result in a *Nco*I restriction site (CCATGG) in front of the start codon. The *Nco*I site was engineered for future purposes. The backward primer 5'-GTCATTACGCCT*GAATTC*C**TCA**TACG-3' contained two mutations that create an *Eco*RI site (italics) immediately behind the TGA stop codon (bold). The *Eco*RI site at the 3' end of the PCR product was cut while the 5' end was left blunt. The fragment was ligated into plasmid pWSK29 (25) digested with *Sma*I and *Eco*RI yielding plasmid pWSKcitM. In pWSKcitM, the open reading frame coding for CitM is downstream of the lac promoter on the vector and the *B. subtilis* ribosomal binding site.
*pWSKcitH*. Open reading frame N15CR coding for CitH was amplified from the same chromosomal DNA. The forward primer 5'- AAAA*AAGCTT*TTGAATAGGGGAGGTCATA
*CC****ATG****G*TTGCCATAC-3' contained three mutations resulting in a *Hind*III site in front of the ribosomal binding site and a *Nco*I site around the start codon. The construction of the *Nco*I site results in the Leu2Val mutation in the primary sequence The backward primer 5'-ATGAATCT*TCTAGA*CACTCATA*G*-*GATCC*TATTGATC-3' was complementary to sequences downstream of the stop codon. Four base changes resulted in the introduction of *Bam*HI and *Xba*I sites (italic) in the PCR product. The PCR product was digested with *Hind*III and *Bam*HI and ligated into plasmid pWSK29 (27) digested with the same two enzymes. In the resulting vector pWSKcitH the citH gene is downstream of the T7 promoter and the *B. subtilis* ribosomal binding site. The base sequences of the inserts in pWSKcitM and pWSKcitH were verified by sequencing the sense strand.

### Transport assays.

*Whole cells*. Cells of *E. coli* harboring plasmids pWSKcitM and pWSKcitH were grown in LB broth as described above and washed twice in 50 potassium phosphate pH 7. Uptake of [1,5-¹⁴C]citrate was measured essentially as described by Lolkema et al. (14). The cells were resuspended in 95 µl of the same buffer with the indicated additions and incubated for 10 min at 37 °C. At time zero 5 µl [1,5-¹⁴C]citrate was added to give a final concentration of 4.5 µM. Uptake was stopped by adding 2 ml of an ice cold 100 mM LiCl solution followed by immediate filtering over 0.45 µm nitrocellulose filters. The filters were washed twice with the LiCl solution and immediately submerged in scintillation fluid to stop any further metabolic activity. The radioactivity retained on the filter was quantified in a liquid scintillation counter.
*Membrane vesicles*. Right-side-out (RSO) membrane vesicles were prepared of the *E. coli* cells harboring plasmids pWSKcitM and pWSKcitH by the osmotic lysis procedure as described by Kaback (11). *E. coli* JM101/pWSKcitM was grown in LB medium and JM109/pWSKcitH in minimal medium supplemented with 20 mM Na-citrate. The membranes were resuspended in 50 mM potassium 1,4-piperazindiethansulfate (Pipes) pH 6.5 at a protein concentration of 15 mg/ml and stored in aliquots in liquid nitrogen. The membrane vesicles were energized by the K-ascorbate/phenazine methosulfate (PMS) electron donor system. The membranes were diluted in 50 mM K-Pipes pH 6.5, and 10 mM K-ascorbate in a total volume of 100 µl and incubated for 5 min at 30 °C under a constant flow of water saturated air. PMS was added at a concentration of 100 µM and the proton motive force was allowed to develop for 1 min, after which [1,5-¹⁴C]citrate was added to a final concentration of 4.5 µM. The uptake was stopped and the samples were processed as described above.

**Materials.** [1,5-¹⁴C]citrate (111 mCi/mmol) was obtained from Amersham Radiochemical Center. Mono potassium phosphate and potassium hydroxide with low Na⁺ content were obtained from Merck. All other chemicals were reagent grade and obtained from commercial sources.

**Data bank submission.** The CitM base sequence has been submitted to the NCBI gene bank and will be accessible under number U62003.

### RESULTS

**Cloning and sequencing of CitM.** The Mg²⁺-dependent citrate carrier of *B. subtilis* that we will term CitM was cloned using conventional techniques. Chromosomal DNA of *B. subtilis* 6GM was partially digested with *Hind*III and the fragments where cloned in the expression vector pINIIIA (8). *Escherichia coli* is an ideal host for the cloning of citrate carriers since it is not capable of taking up citrate but metabolizes it readily in the citric acid cycle. The expression vector containing the chromosomal fragments were transformed to *E. coli* JM 101 and grown on citrate indicator plates (Simmons agar). A number of blue colonies, indicative of citrate uptake and metabolism, were assayed for their ability to take up citrate in the presence and absence of 10 mM MgCl₂. A clone (plasmid pM5) was selected that showed no uptake activity in the absence of Mg²⁺ and a high uptake in the presence of Mg²⁺ (not shown). Restriction analysis of pM5 revealed the presence of a 5.6 kb insert containing 3 additional *Hind*III sites. A 2.8 kbase *Eco*RI fragment of pM5 was subcloned into the *Eco*RI site of pINIIIA. Only one of the two orientations of the insert (plasmid pM6) showed Mg²⁺-dependent citrate uptake activity revealing the presence of the gene on the fragment and showing that the gene is expressed from the tandem promoter on pINIIIA. Further subcloning of pM6 resulted in the loss of the citrate utilizing phenotype. pM6 was used to subclone the 0.9 kb *Eco*RI/*Hind*III fragment (pSK0.9) and the 1.8 kb *HindIII*- *EcoRI* fragment (pSK1.8) in pBluescript. The two subclones were used to make sets of nested deletions to determine the nucleotide sequence of the gene. Both subclones were sequenced in both directions. Reconstruction of the base sequence of the insert on pM6 from the sequences of the two subclones revealed an open reading frame downstream of the promoter region on the vector with a length of 1302 bp and starting with a GTG codon (Fig 1). The length of the ORF conforms to the expected length of a gene coding for a bacterial secondary transporter. A putative ribosomal binding site is located upstream of the GTG start codon that shows extensive similarity to the 3' end of *B. subtilis* 16S rRNA. Neither a clear promoter sequence nor a terminator sequence were found upstream and downstream of the ORF, respectively. The complete base sequence has been deposited in the NCBI gene bank and will be available under accession number U63002.

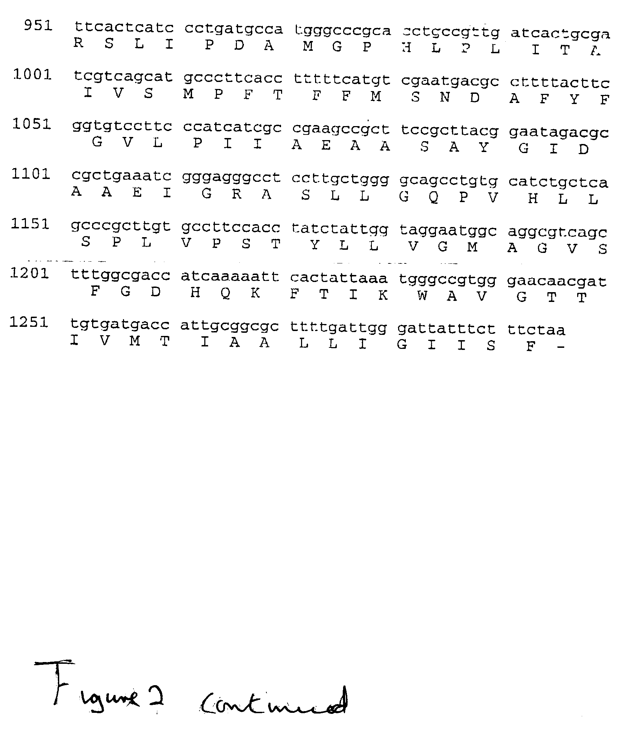

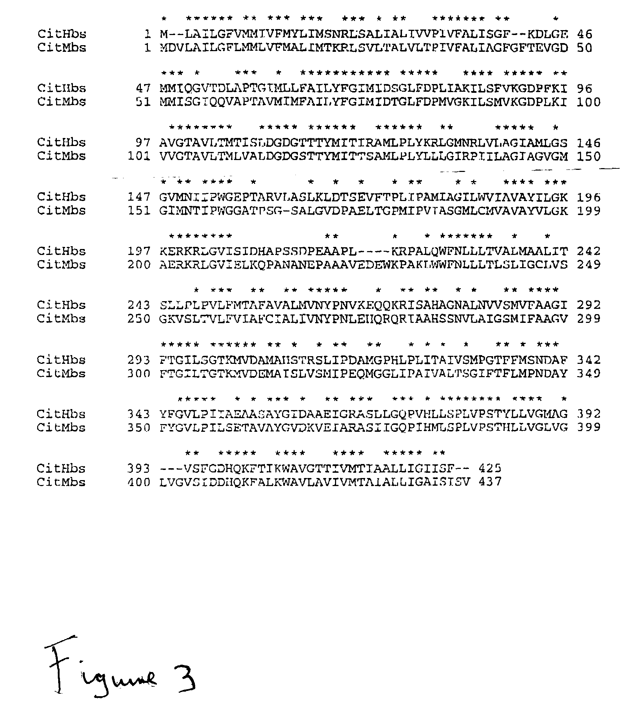

A non redundant search of the available gene banks revealed an ORF of 1278 bp with 60% of base identity with the cloned gene. The ORF indicated as N15CR was detected in the bglS-katB intergenic region on the genome of *B. subtilis* 168. The ORF (Fig 2) starts with an ATG codon and is preceded by a ribosomal binding site. No clear promoter region could be detected upstream. The alignment (Fig 3) of the CitM sequence with the N15CR ORF shows that the ATG codon that lies in between the ribosomal binding site and the putative CitM GTG start codon is unlikely to function as the initiator of translation (Fig. 2).

The presence of the citM gene and the N15CR ORF on the genome of *B. subtilis* 6GM was confirmed by PCR and Southern hybridization. DNA probes were prepared by amplifying the first 487 bp of citM and the last 463 bp of N15CR by PCR using chromosomal DNA of *B. subtilis* 6GM as the template. The probes were selected such that they contained no *Hind*III restriction sites. The PCR resulted in distinct DNA fragments of the expected length. The nucleotide analog DIG-dUTP was incorporated into the fragments for the use as probes in Southern blotting. The two probes detected unique, but different fragments of *B. subtilis* 6GM genomic DNA restricted with *Hind*III. Both the fragments were of the expected length. Plasmids pWSKcitM and pWSKcitH that contain the citM gene and ORF N15CR (see below) hybridized exclusively with the citM and N13CR probes, respectively, in spite of the high sequence identity between the two genes. The lack of cross-reaction reflects the high stringency of the hybridization and washing conditions. Under these conditions the two probes did not detect similar genes on the chromosome of *E. coli* and the thermophilic *Bacillus* species *B. stearothermophilus*.

**Primary sequence analysis.** Translation of the base sequences of the cloned ORF and the corresponding ORF from the *B. subtilis* gene bank results in two proteins with approximately 60 % amino acid sequence identity and an additional 18 % of similar residues. The amino acid composition of the two proteins is typical for integral membrane proteins with an average hydrophobicity of 0.51 and 0.47 on the normalized scale of Kyte (5), respectively. The hydropathy profiles of the two sequences are remarkably similar. Significant differences show up only in the region from position 125 to 145 and to a lesser extent in the region from position 310 to 330. In both regions, CitM is the more hydrophobic sequence. The high similarity of the two sequences suggests a similar folding in the membrane. Secondary structure prediction (6) results in 12 membrane spanning, presumably α-helical, regions both for the CitM protein and the protein coded by the N15CR ORF. Assuming similar folds for the two proteins, merging of the two predictions results in 12 transmembrane segments, interspaced by 11 loops. The respective nucleic acid sequences corresponding to the 12 transmembrane segements and the 11 loops relate more or less to the sequence as shown in figure 1 from position 7 to 71, or from 72 to 77, or from 78 to 144, or from 145 to 182, or from 183 to 243, or from 244 to 272, or from 273 to 338, or from 339 to 398, or from 399 to 458, or from 459 to 536, or from 537 to 596, or from 597 to 704, or from 705 to 773, or from 774 to 848, or from 849 to 923, or from 924 to 965, or from 966 to 1037, or from 1038 to 1055, or from 1056 to 1109, or from 1110 to 1148, or from 1149 to 1202, or from 1203 to 1228, or from 1229 to 1316, respectively. It can be expected that within these transmembrane fragments and loops lay topology requirements and active sites which, when modified via recombinant technology, will alter the metal specificity of the encoded citrate transporter protein.

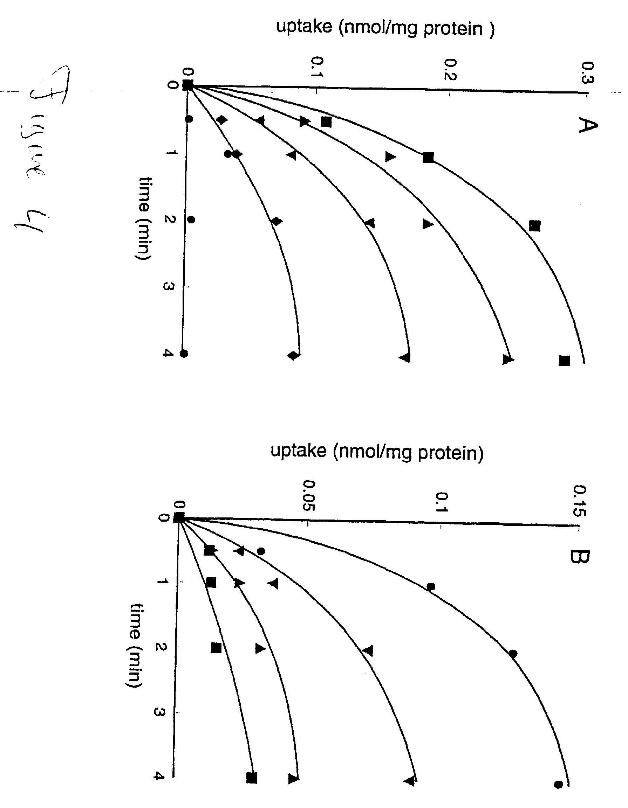

**Substrate specificity.** The citM gene and the N15CR ORF (CitH gene) were amplified by PCR using *B. subtilis* chromosomal DNA as template. The citM fragment was cloned downstream of the lac promoter region on plasmid pWSK29 a low copy pBluescript derivative (25) yielding plasmid pSKcitM. The N15CR ORF was cloned behind the T7 promoter on the same plasmid yielding pWSKcitH. The sequence of the cloned PCR fragments was verified in one direction and found to be identical to the base sequence of the original ORFs except for the second codon of citH which now codes Val instead of Leu (see the Methods section for details).
Plasmid pWSKcitM was transformed to *E. coli* JM101 and plasmid pWSKcitH to *E.coli* JM109(DE3) a strain that contains a chromosomal copy of T7 polymerase and the cells were plated on Simmons agar indicator plates. Surprisingly, both plasmids conferred the citrate utilizing phenotype. Apparently, the N15CR ORF codes for a citrate transporter as well. We will term this transporter CitH. Figure 4 shows the uptake of citrate in cells harboring plasmids pWSKcitM (A) and pWSKcitH (B) in the presence of different concentrations of Mg²⁺. Citrate uptake activity in cells expressing CitM is completely absent in the absence of Mg²⁺-ions. The uptake activity increases with increasing Mg²⁺ concentrations consistent with the Mg²⁺-citrate complex being the substrate of the carrier (2). In marked contrast, cells harbouring plasmid pWSKCitH expressing the citrate carrier coded by ORF N15CR (citH) readily take up citrate in the absence of Mg²⁺. Increasing concentrations of Mg²⁺ in the assay buffer result in decreasing uptake rates. Apparently, the substrate of CitH is free citrate as is the case for the Na⁺ and H⁺-dependent citrate carriers of *K. pneumoniae* (23,24) and the membrane potential generating citrate carrier of *L. mesenteroides* (16).

**Co-ion specificity.** The involvement of Na⁺ ions in the uptake of citrate by CitM and CitH was investigated by measuring the uptake of citrate in *E. coli* strains JM101/pWSKcitM and JM109(DE3)/pWSKcitH in the presence and absence of 10 mM NaCl. Prior to the experiments the cells were washed three times in large volumes of potassium phosphate pH 7 containing especially low amounts of Na⁺.

The residual Na⁺ ion concentration was at most a few µM. Furthermore, the uptake experiments were performed in plastic tubes to prevent Na⁺ contamination from glassware. For both transporters the uptake of citrate was not significantly different in the presence or absence of NaCl indicating that Na⁺ is not a co-ion for CitM nor CitH (not shown).
Studies with membrane vesicles prepared from *B. subtilis* cells have demonstrated that the Mg²⁺ dependent citrate transporter CitM is a secondary transporter that is driven by the proton motive force (2). The high similarity between CitM and CitH suggests that the same is true for CitH. The energy coupling mechanism of both cloned transporters was investigated by preparing right-side-out membrane vesicles of *E. coli* cells expressing CitM or CitH. The membranes were energized by the ascorbate/PMS electron donor system. In the presence of a proton motive force, both transporters accumulated citrate indicating cotransport by CitM of the Mg²⁺/citrate complex and protons and by CitH of citrate and protons. In the presence of the K⁺ ionophore valinomycin which results in the dissipation of the membrane potential component of the proton motive force the uptake activity was slightly less .In the presence of nigericin, a K⁺/H⁺ antiporter, which dissipates the pH gradient across the membrane and results in a proton motive force that is solely composed of the membrane potential, significant uptake above background is still observed. It is concluded that both CitM and CitH are electrogenic transporters that translocate net positive charge into the cells. The complex between citrate and Mg²⁺ is monovalent anionic (MgCit⁻) and, therefore, CitM cotransports at least 2 protons per Mg²⁺/citrate complex. Electrogenic transport by CitH indicates cotransport of at least 3 or 4 protons depending on translocation of Hcit²⁻ or cit³⁻, respectively.

### DISCUSSION

The cloning and sequencing of the Mg²⁺ dependent citrate carrier of *B. subtilis* CitM let to the surprising finding of a second citrate carrier in *B. subtilis* CitH for which the gene was deposited in the databanks as open reading frame N15CR. The two transporters share common properties at different levels: (i) the coding genes are 61.5% homologous and in the primary sequence alignment about 60% residues are identical, (ii) the transporters function as electrogenic proton symporters and (iii) the genes coding for the transporters are present on the chromosome of *B. subtilis* and the genes were not found in *E. coli* and *B. stearothermophilus*. The most striking difference between the two transporters is that CitM transports the Mg²⁺/citrate complex while CitH transports free citrate in the uncomplexed state and is hampered by the presence of Mg². In *B. subtilis*, CitM is induced by citrate in the medium and the absence of citrate uptake by membrane vesicle in the presence of EDTA indicates that CitM is the only transporter induced under these conditions (2). The experiments with the cloned transporter in *E. coli* emphasize that CitM transports only citrate in the Mg²⁺ complexed form. Therefore, CitH is not induced under the same conditions in *B. subtilis*. Open reading frame N15CR that codes for CitH lies in between the genes *bglS* and *katB* on the *B. subtilis* genome. It is coded in the opposite direction relative to these two genes. Gene *bglS* codes for lichanase, an exported enzyme, that hydrolyses mixed linked β-glucans (27) and *katB* codes for a catalase involved in sporulation (13). The *citH* gene is not preceded by a known promoter sequence and does not seem to be part of a operon structure suggesting that the gene is silent. On the other hand, the gene is preceded by a ribosomal binding site and results in a functional transporter when expressed from a heterologous promoter. Either the gene has become silent only very recently on a evolutionary time scale or, more likely, the gene is expressed under special, unknown conditions using an unknown promoter sequence.
The high sequence identity of CitM and CitH suggests that the binding sites for the Mg²⁺/citrate complex and citrate are not very different. The two transporters may be very suitable for the construction of chimeric proteins to localize the substrate binding site in the primary sequence. The successful construction of active chimeras can be tested on citrate indicator plates and the Mg²⁺-dependency provides an easy way to discriminate between the activity of the two transporters. We constructed one chimera by making use of a conserved *Stu*I restriction site in the two genes around position 490. The hybrid protein consisted of the N-terminal CitM fragment and the C-terminal CitH fragment. We are now in the process of constructing a series of chimeras by introducing unique restriction sites at selected sites in the *citM* and *citH* genes.

### REFERENCES

1. **Bandell, M., Ansanay, V., Rachidi, N., Dequin, S. and Lolkema, J.S.** 1996. The membrane potential generating citrate and malate transporters of lactic acid bacteria are homologous protein. The 2-hydroxy-carboxylate family of transporters. J. Biol. Chem., submitted
2. **Bergsma, J. and Konings, W.N.** 1983 The properties of citrate transport in membrane vesicles from *Bacillus subtilis.* Eur. J. Biochem. **134**:151-156.
3. **Brynhildsen, L. and Rosswall, T.** 1989. Effects of cupper, magnesium and zinc on the decomposition of citrate by *Klebsiella* sp. Appl. Environm. Microbiol. **55**:1375-1379.
4. **David, S., M. E. van der Rest, A. J. M. Driessen, G. Simons, and W. de Vos** 1990 Nucleotide sequence and expression in *Escherichia coli* of the *Lactococcus lactis* citrate permease gene. J. Bacteriol. **172**:5789-5794.
5. **Eisenberg, D.** 1984. Three-dimensional structure of membrane and surface proteins. Ann. Rev. Biochem. **53**:595-623.
6. **Eisenberg, D., Schwarz, E., Komarony, M. and Wall, R.** 1984 Analysis of membrane and surface protein sequences with the hydrophobic moment plot. J. Mol. Biol. **179**:125-142.
7. **Francis, A.J., Dodge, C.J. and Gillow, J.B.** 1992. Biodegradation of metal citrate complexes and implications for toxic-metal mobility. Nature **356**:140-142.
8. **Inouye, H.** 1983. Experimental manipulation of gene expression. Academic Press Inc., New York.
9. **Ishiguro, N., H. Izawa, M. Shinagawa, T. Shimamoto, and T. Tsuchiya** 1992. Cloning and nucleotide sequence of the gene (*citC*) encoding a citrate carrier from several *Salmonella* serovars. J. Biol. Chem. **267**:9559-9564.
10. **Joshi-Tope, G. and Francis, A.J.** 1995. Mechanisms of biodegradation of metal-citrate complexes by *Pseudomonas fluorescens.* J. Bacteriol. **177**:1989-1993.
11. **Kaback, H.R.** 1971. Bacterial membranes. Methods Enzymol. **22**:99-120.
12. **Konings, W.N., Lolkema, J.S. and Poolman, B.** 1995. The generation of metabolic energy by solute transport. Arch. Microbiol. **164**:235-242.
13. **Loewen, P.C.** 1989. Genetic mapping of katB, a locus that affects catalase 2 levels in Bacillus subtilis. Can J Microbiol **35**:807-810.
14. **Lolkema, J. S., H. Enequist, and M. E. van der Rest** 1994. Transport of citrate catalyzed by the sodium-dependent citrate carrier of *Klebsiella pneumoniae* is obligatory coupled to the transport of two sodium ions. Eur. J. Biochem. **220**:469-475.
15. **Lolkema, J.S., Poolman B., and Konings, W.N.** 1996. Secondary transporters and metabolic energy generation in bacteria. In: Handbook of Biological Physics. Ed. Konings, W.N., Kaback, H.R. and Lolkema, J.S. Elsevier Science, Amsterdam.
16. **Marty-Teysset, C., J. S. Lolkema, P. Schmitt, C. Divies, and W. N. Konings** 1995. Membrane potential generating transport of citrate and malate catalyzed by CitP of *Leuconostoc mesenteroides.* J. Biol. Chem. **270**:25370-25376.
17. **Marty-Teysset, C., Posthuma, C., Lolkema, J.S., Schmitt, P., Divies, C. and Konings, W.N.** 1996. Proton motive force generation by citrolactic fermentation in *Leuconostoc mesenteroides*. J. Bacteriol. **178**:2178-2185.
18. **Poolman, B., Konings. W.N.** 1993. Secondary solute transport in bacteria. Biochim. Biophys. Acta **1183**:5-39.
19. **Pos, K. M. and P. Dimroth** 1996. Functional properties of the purified Na⁺-dependent citrate carrier of *Klebsiella pneumoniae*: Evidence for asymmetric orientation of the carrier protein in proteoliposomes. Biochemistry **35**:1018-1026.
20. **Ramos, A., B. Poolman, H. Santos, J. S. Lolkema, and W. N. Konings** 1994. Uniport of anionic citrate and proton consumption in *Leuconostoc oenos.* J. Bacteriol. **176**:4899-4905.
21. **Sambrook, J., Fritz, E.F. and Maniatis, T.** 1989. Molecular cloning: a laboratory manual. Cold Spring Harbor Laboratory Press, Cold Spring Harbor, New York.
22. **Van der Rest, M. E., E. Schwarz, D. Oesterhelt, and W. N. Konings** 1990. DNA sequence of a citrate carrier of *Klebsiella pneumoniae*. *Eur. J. Biochem.* **189**:401-407.
23. **Van der Rest, M. E., T. Abee, D. Molenaar, and W. N. Konings** 1991. Mechanism and energetics of a citrate transport system of *Klebsiella pneumoniae*. Eur. J. Biochem. **195**:71-77.
24. **Van der Rest, M. E., R. M. Siewe, T. Abee, E. Schwarz**, **D. Oesterhelt, and W. N. Konings** 1992. Nucleotide sequence and functional properties of a sodium-dependent citrate transport system from *Klebsiella pneumoniae*. J. Biol. Chem. **267**:8971-8976.
25. **Wang R.F. and Kushner S.R.** 1991. Construction of versatile low-copy-number vectors for cloning, sequencing and gene expression in Escherichia coli. Gene **100**:195-199.
26. **Willeke, K., Gries, E-M. and Oehr, P.** 1973. Coupled transport of citrate and magnesium in *Bacillus subtilis*. J. Biol. Chem. **248**:807-814.
27. **Wolf, M., Geczi, A., Simon, O. and Borriss, R.** 1995. Genes encoding xylan and β-glucan hydrolysing enzymes in *B. subtilis*: characterization, mapping and construction of strains deficient in lichanase, cellulase and xylanase. Microbiology **141**:281-290.

### Figure legends

Figure 1
   Nucleotide sequence and corresponding amino acide sequence of the CitM gene.
Figure 2
   Nucleotide sequence and corresponding amino acide sequence of the CitH gene.
Figure 3
   Alignment of amino acid sequences of the CitM and CitH genes.
Figure 4
   Mg²⁺-ion dependence of the uptake activity of CitM (A) and CitH (B). [1,5¹⁴C]citrate uptake by *E. coli* JM101/pSKcitM (A) and *E. coli* JM109(DE3)/pSKcitH (B) was measured in 50 mM potassium phosphate pH 7 supplemented with 0 (●), 0.5 (◆), 1 ( 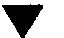 ), 5 (▲) and 10 (■) mM MgCl₂. The cell protein concentrations were 0.6 (A) and 1.2 (B) mg/ml.

## Claims

1. A recombinant nucleic acid comprising a nucleic acid sequence as shown in figure 1 or functional fragments or functional derivatives thereof or a recombinant nucleic acid which is at least 65% homologous to the sequence shown in figure 1.

2. A recombinant nucleic acid consisting of a gene encoding a citrate transporter protein and having the nucleic acid sequence of figure 2 or functional fragments or functional derivatives thereof.

3. A nucleic acid according to claim 1 or 2 which encodes a citrate transporter protein which transports free citrate.

4. A nucleic acid according to claim 1 or 2 which encodes a citrate transporter protein which transports a metal-citrate complex.

5. A nucleic acid according to any one of claims 1 to 4 in which a nucleic acid sequence corresponding to the sequence as shown in figure 1 from position 7 to 71, or from 72 to 77, or from 78 to 144, or from 145 to 182, or from 183 to 243, or from 244 to 272, or from 273 to 338, or from 339 to 398, or from 399 to 458, or from 459 to 536, or from 537 to 596, or from 597 to 704, or from 705 to 773, or from 774 to 848, or from 849 to 923, or from 924 to 965, or from 966 to 1037, or from 1038 to 1055, or from 1056 to 1109, or from 1110 to 1148, or from 1149 to 1202, or from 1203 to 1228, or from 1229 to 1316, has been modified.

6. A vector comprising a nucleic acid according to any one of claims 1 to 5.

7. A host cell comprising a vector according to claim 6.

8. A host cell comprising a nucleic acid according to any one of claims 1 to 5.

9. A host cell expressing a protein encoded by a nucleic acid according to any one of claims 1 to 5.

10. A citrate transporter protein obtainable by growing a host cell according to any one of claims 7 to 9.

11. A process for recovering metal comprising using a host cell according to any one of claims 7 to 9.

12. A process for recovering metal comprising using a protein according to claim 10.

13. Use of a microorganism comprising a nucleotide sequence as shown in figure 1 or 2 or comprising a nucleotide sequence which is at least 65% homologous to the sequence shown in figure 1 or 2 for the industrial recovery of metal.
